(19) Europäisches Patentamt
European Patent Office
Office européen des brevets

(11) **EP 3 183 032 B1**

(12) **EUROPÄISCHE PATENTSCHRIFT**

(45) Veröffentlichungstag und Bekanntmachung des Hinweises auf die Patenterteilung:
**20.02.2019 Patentblatt 2019/08**

(21) Anmeldenummer: **15800873.0**

(22) Anmeldetag: **26.11.2015**

(51) Int Cl.:
**A61N 1/36** *(2006.01)*          **A61N 1/05** *(2006.01)*
**A61N 1/34** *(2006.01)*

(86) Internationale Anmeldenummer:
**PCT/EP2015/077797**

(87) Internationale Veröffentlichungsnummer:
**WO 2016/083516 (02.06.2016 Gazette 2016/22)**

(54) **VORRICHTUNG ZUR EFFEKTIVEN INVASIVEN NEUROSTIMULATION MITTELS VARIIERENDER REIZSEQUENZEN**

DEVICE FOR EFFECTIVE INVASIVE NEUROSTIMULATION BY MEANS OF VARYING STIMULUS SEQUENCES

DISPOSITIF DE NEUROSTIMULATION INVASIVE EFFICACE À L'AIDE DE SÉQUENCES D'EXCITATION VARIABLES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.11.2014 DE 102014117429**

(43) Veröffentlichungstag der Anmeldung:
**28.06.2017 Patentblatt 2017/26**

(73) Patentinhaber: **Forschungszentrum Jülich GmbH 52425 Jülich (DE)**

(72) Erfinder:
• **TASS, Peter Alexander**
  **83684 Tegernsee (DE)**
• **ZEITLER, Magteld**
  **6581 JL Malden (NL)**

(74) Vertreter: **Vossius & Partner**
  **Patentanwälte Rechtsanwälte mbB**
  **Siebertstrasse 3**
  **81675 München (DE)**

(56) Entgegenhaltungen:
**DE-A1-102010 016 461     US-A1- 2006 069 415**

**Beschreibung**

Die Erfindung betrifft eine Vorrichtung zur effektiven invasiven Neurostimulation mittels variierender Reizsequenzen.

Bei Patienten mit neurologischen oder psychiatrischen Erkrankungen, z. B. Morbus Parkinson, essentiellem Tremor, Epilepsie, Funktionsstörungen nach Schlaganfall, Dystonie oder Zwangserkrankungen, sind Nervenzellverbände in umschriebenen Bereichen des Gehirns, z. B. des Thalamus und der Basalganglien krankhaft, z. B. übersteigert synchron, aktiv. In diesem Fall bildet eine große Anzahl von Neuronen synchron Aktionspotentiale aus, d. h., die beteiligten Neuronen feuern übermäßig synchron. Beim Gesunden hingegen feuern die Neuronen in diesen Hirngebieten qualitativ anders, z. B. auf unkorrelierte Weise.

Beim Morbus Parkinson verändert die pathologisch synchrone Aktivität die neuronale Aktivität in anderen Hirngebieten, z. B. in Arealen der Großhirnrinde wie dem primär motorischen Cortex. Dabei zwingt die pathologisch synchrone Aktivität im Bereich des Thalamus und der Basalganglien beispielsweise den Großhirnrindenarealen ihren Rhythmus auf, so dass schließlich die von diesen Arealen gesteuerten Muskeln pathologische Aktivität, z. B. ein rhythmisches Zittern (Tremor), entfalten.

Zur Behandlung von medikamentös nicht hinreichend behandelbaren Parkinsonpatienten wird die tiefe Hirnstimulation eingesetzt. Hierbei werden Tiefenelektroden in speziellen Hirngebieten, z. B. im Nucleus subthalamicus, implantiert. Zur Linderung der Symptome wird über die Tiefenelektroden eine elektrische Reizung durchgeführt. Bei der Standard-Hochfrequenz-Stimulation zur Behandlung der Parkinsonschen Erkrankung wird eine sogenannte Hochfrequenz-Dauerreizung bei Frequenzen von über 100 Hz durchgeführt. Diese Behandlungsart hat keine lang anhaltenden therapeutischen Effekte (vgl. P. Temperli, J. Ghika, J.-G. Villemure, P. Burkhard, J. Bogousslavsky, and F. Vingerhoets: How do parkinsonian signs return after discontinuation of subthalamic DBS? Neurology 60, 78 (2003)). Mit weniger Reizstrom kommt die "Coordinated Reset"-Stimulation (CR-Stimulation) aus, die obendrein lang anhaltende therapeutische Effekte haben kann (P. A. Tass, L. Qin, C. Hauptmann, S. Doveros, E. Bezard, T. Boraud, W. G. Meissner: Coordinated reset neuromodulation has sustained after-effects in parkinsonian monkeys. Annals of Neurology 72, 816-820 (2012); I. Adamchic, C. Hauptmann, U. B. Barnikol, N. Pawelcyk, O.V. Popovych, T. Barnikol, A. Silchenko, J. Volkmann, G. Deuschl, W. Meissner, M. Maarouf, V. Sturm, H.-J. Freund, P. A. Tass: Coordinated Reset Has Lasting Aftereffects in Patients with Parkinson's Disease. Movement Disorders 29, 1679 (2014)).

Bei anderen Erkrankungen, z. B. bei medikamentös nicht ausreichend behandelbaren Epilepsien, werden neben Tiefenelektroden auch andere, z. B. epikortikale oder epidurale Elektroden, implantiert. Bei weiteren Erkrankungen, z. B. chronischen Schmerz-Syndromen, ist es üblich, nicht nur mittels Tiefenelektroden im Gehirn, sondern auch mittels z. B. epiduralen Elektroden das Rückenmark zu reizen. Anders als die CR-Stimulation haben die meisten anderen Stimulationsarten keine lang anhaltenden therapeutischen Effekte.

Therapeutische Effekte können auch durch direkte Stimulation des Hirngewebes bzw. Rückenmarks mit Licht, z. B. über implantierte Lichtleiter, erzielt werden. Hierbei können auch unterschiedliche raum-zeitliche Stimulationsmuster, wie z. B. die CR-Stimulation, zur Anwendung kommen.

Obgleich die tiefe Hirnstimulation mittels invasiver CR-Stimulation lang anhaltende therapeutische Effekt ermöglicht, hat dieser Ansatz relevante Limitationen:

(a) Typischerweise wird zur Reduktion des Stromeintrags intermittent, d. h. mittels einer Vielzahl von Stimulationsepochen gereizt. Die intermittente Stimulation kann die Nebenwirkungsrate reduzieren und den gesamten Stromverbrauch einschränken. Da ein deutlich verringerter Stromverbrauch die Verwendung einer deutlich kleineren Batterie bzw. eines entsprechenden Akkus ermöglicht, kann auf diese Weise ermöglicht werden, besonders kleine, (bzgl. OP-Trauma und Infektionsrisiko) schonende Implantate zu verwenden. Die Wirkung der bisherigen CR-Stimulation schwankt von Stimulationsepoche zu Stimulationsepoche zu stark, d. h., es gibt zu viele Stimulationsepochen mit nicht hinreichend ausgeprägtem Effekt. Mit anderen Worten ist die Stimulationswirkung in relevantem Ausmaß von den Anfangsbedingungen des Organismus bzw. Nervensystems abhängig, bei denen die Stimulation gestartet wird. Wird z. B. bei der einen Stimulationsepoche ein sehr guter Effekt erzielt, so ist dieser bei einer nächsten Stimulationsepoche eher unbefriedigend. Um die weniger effektiven Stimulationsepochen zu kompensieren, ist eine größere Zahl von Stimulationsepochen nötig, um einen guten therapeutischen Effekt aufzubauen.

(b) Bei der bisherigen Form der CR-Stimulation hängt der Stimulationserfolg zu stark von der Reizintensität ab. Unterschiedliche Faktoren können die Reizstärke relevant modifizieren. Z. B. kann die effektive Reizintensität, die an der neuronalen Zielpopulation tatsächlich ankommt, durch eine Narbenbildung um implantierte Tiefenelektroden absinken.

(c) Die Reizstärke ist ganz generell in Relation zu charakteristischen Kenngrößen des zu stimulierenden Systems, also des Körpers bzw. Nervensystems zu sehen. Da diese Kenngrößen (z. B. bestimmte Ionenkonzentrationen, Flüssigkeitsvolumina, Hormonkonzentrationen etc.) schwanken und z. B. ausgeprägten tageszeitlichen Schwankungen unterworfen sind, sollte eine optimale Reizstärke entweder entsprechend nachgeregelt werden, oder es sollte ein Stimulationsverfahren verwandt werden, dessen Stimulationseffekte möglichst unabhängig von diesen Schwankungen sind.

Zusammengefasst ist die Wirkung der bisher verwandten CR-Stimulation nicht hinreichend robust gegenüber Schwankungen der Reizintensität sowie gegenüber charakteristischen Kenngrößen des zu stimulierenden Organismus bzw. Nervensystems (zu Beginn der Stimulation sowie im Verlauf der Stimulation) und insbesondere schwankt die Wirkung der CR-Stimulation von Stimulationsepoche zu Stimulationsepoche zu stark, d. h., es gibt zu viele Stimulationsepochen mit geringem Effekt.

Herkömmliche Vorrichtungen zur invasiven Neurostimulation sind in den Dokumenten DE 10 2010 016 461 A1 und US 2006 / 0 069 415 A1 beschrieben.

Der Erfindung liegt die Aufgabe zugrunde, eine Vorrichtung anzugeben, die es ermöglicht, über einen weiten Intensitätsbereich verbesserte und insbesondere lang anhaltende therapeutische Effekte zu erzielen.

Die der Erfindung zugrunde liegende Aufgabenstellung wird durch die Merkmale der unabhängigen Ansprüche gelöst. Vorteilhafte Weiterbildungen und Ausgestaltungen der Erfindung sind in den Unteransprüchen angegeben.

Die Erfindung wird nachfolgend in beispielhafter Weise unter Bezugnahme auf die Zeichnungen näher erläutert. In diesen zeigen:

Fig. 1      eine schematische Darstellung einer Vorrichtung zur Unterdrückung einer krankhaft synchronen und oszillatorischen neuronalen Aktivität und insbesondere zur Desynchronisierung von Neuronen mit einer krankhaft synchronen und oszillatorischen Aktivität gemäß einer ersten Ausgestaltung;

Fig. 2A      eine schematische Darstellung einer CR-Stimulation mit schnell variierenden Reizsequenzen;

Fig. 2B      eine schematische Darstellung einer CR-Stimulation mit langsam variierenden Reizsequenzen;

Fig. 3      eine schematische Darstellung einer Vorrichtung zur Unterdrückung einer krankhaft synchronen und oszillatorischen neuronalen Aktivität und insbesondere zur Desynchronisierung von Neuronen mit einer krankhaft synchronen und oszillatorischen Aktivität gemäß einer zweiten Ausgestaltung;

Fig. 4      eine schematische Darstellung einer Vorrichtung zur elektrischen Stimulation von Neuronen mit einer krankhaft synchronen und oszillatorischen neuronalen Aktivität; und

Fig. 5 bis 7      Diagramme mit Simulationsergebnissen für schnell und langsam variierende CR-Stimulationen.

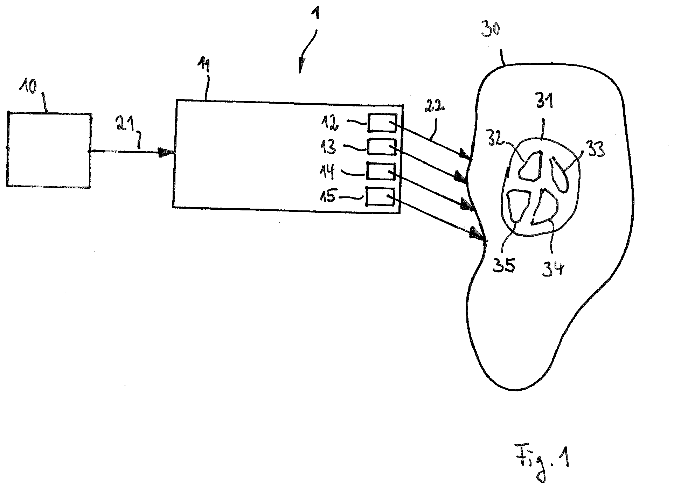

In Fig. 1 ist schematisch eine Vorrichtung 1 zur Stimulation von Neuronen mit einer krankhaft synchronen und oszillatorischen neuronalen Aktivität dargestellt. Die Vorrichtung 1 besteht aus einer Steuereinheit 10 und einer Stimulationseinheit 11, die eine Mehrzahl von Stimulationselementen aufweist. In Fig. 1 sind beispielhaft vier Stimulationselemente 12, 13, 14 und 15 dargestellt. Die Stimulationseinheit 11 kann selbstverständlich aber auch eine andere Zahl von Stimulationselementen aufweisen. Im Fall von elektrischer Stimulation kann es sich bei den Stimulationselementen 12 bis 15 z. B. um Stimulationskontaktflächen einer oder mehrerer Elektroden zur Applikation elektrischer Reize handeln. Falls optisch stimuliert wird, können z. B. Lichtleiter als Stimulationselemente 12 bis 15 eingesetzt werden, um das neuronale Gewebe an den gewünschten Stellen mit Lichtreizen zu stimulieren.

Während des Betriebs der Vorrichtung 1 führt die Steuereinheit 10 eine Steuerung der Stimulationseinheit 11 durch. Dazu erzeugt die Steuereinheit 10 Steuersignale 21, die von der Stimulationseinheit 11 entgegengenommen werden.

Die Stimulationseinheit 11 wird operativ in den Körper des Patienten implantiert und erzeugt anhand der Steuersignale 21 Reize 22, insbesondere elektrische und/oder optische Reize 22, die dem Gehirn und/oder Rückenmark 30 des Patienten verabreicht werden. Die Reize 22 sind dazu ausgelegt, bei einer Verabreichung an den Patienten die krankhaft synchrone und oszillatorische neuronale Aktivität zu unterdrücken und insbesondere die Neuronen mit der krankhaft synchronen und oszillatorischen Aktivität zu desynchronisieren.

Die Steuereinheit 10 kann eine nicht-invasive Einheit sein, d. h., während des Betriebs der Vorrichtung 1 befindet sie sich außerhalb des Körpers des Patienten und wird nicht operativ in den Körper des Patienten implantiert.

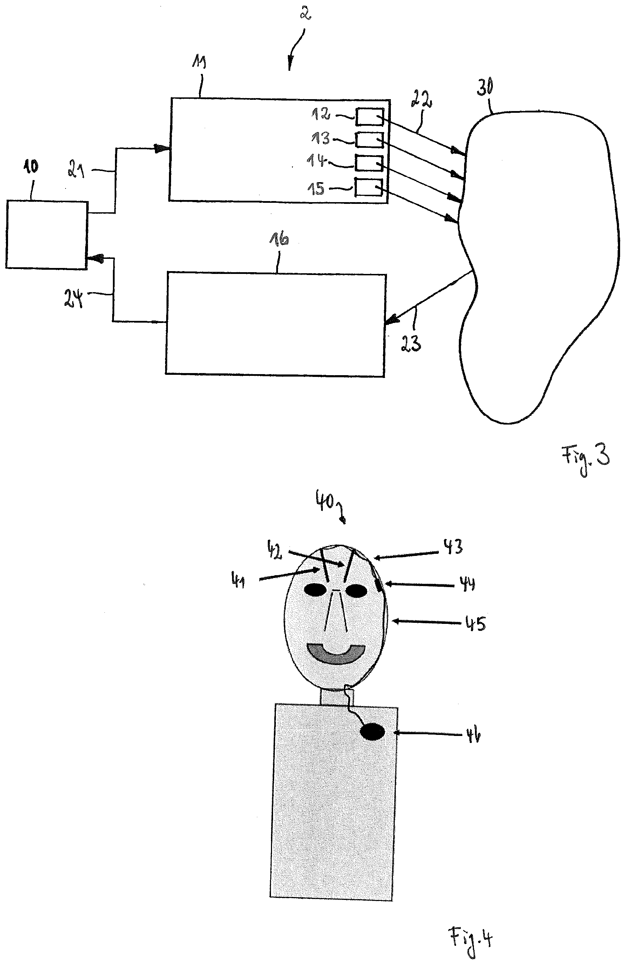

Die Vorrichtung 1 und die weiter unten, im Zusammenhang mit Fig. 3 beschriebene Vorrichtung 2 können insbesondere zur Behandlung von neurologischen oder psychiatrischen Erkrankungen eingesetzt werden, z. B. Morbus Parkinson, essentiellem Tremor, Tremor infolge von Multipler Sklerose sowie anderen pathologischen Tremores, Dystonie, Epilepsie, Depression, Bewegungsstörungen, Kleinhirnerkrankungen, Zwangserkrankungen, Demenzerkrankungen, Morbus Alzheimer, Tourette-Syndrom, Autismus, Funktionsstörungen nach Schlaganfall, Spastik, Tinnitus, Schlafstörungen, Schizophrenie, Reizdarm-Syndrom, Suchterkrankungen, Borderline-Persönlichkeitsstörung, Aufmerksamkeits-Defizit-Syndrom, Aufmerksamkeits-Defizit-Hyperaktivitäts-Syndrom, Spielsucht, Neurosen, Fresssucht, Magersucht, Essstörungen, Burnout-Syndrom, Fibromyalgie, Migräne, Cluster-Kopfschmerz, allgemeiner Kopfschmerz, Neuralgie, Ataxie, Tic-Störung oder Hypertonie, sowie weiteren Erkrankungen, die durch krankhaft gesteigerte neuronale Synchronisation gekennzeichnet sind.

Die vorstehend genannten Krankheiten können durch eine Störung der bioelektrischen Kommunikation von Neuronenverbänden, die in spezifischen Schaltkreisen zusammengeschlossen sind, verursacht werden. Hierbei generiert eine Neuronenpopulation anhaltend krankhafte neuronale Aktivität und möglicherweise eine damit verbundene krankhafte Konnektivität (Netzwerkstruktur). Dabei bildet eine große Anzahl von Neuronen synchron Aktionspotentiale aus, d. h., die beteiligten Neuronen feuern übermäßig synchron. Hinzu kommt, dass die kranke Neuronenpopulation eine oszillatorische neuronale Aktivität aufweist, d. h., die Neuronen feuern rhythmisch. Im Fall von neurologischen oder psychiatrischen Erkrankungen liegt die mittlere Frequenz der krankhaften rhythmischen Aktivität der betroffenen Neuronenverbände etwa im Bereich von 1 bis 30 Hz, kann aber auch außerhalb dieses Bereichs liegen. Bei gesunden Menschen feuern die Neuronen hingegen qualitativ anders, z. B. auf unkorrelierte Weise.

In Fig. 1 ist die Vorrichtung 1 während einer CR-Stimulation dargestellt. Im Gehirn und/oder Rückenmark 30 des Patienten weist mindestens eine Neuronenpopulation 31 eine wie vorstehend beschrieben krankhaft synchrone und oszillatorische neuronale Aktivität auf. Die Stimulationseinheit 11 stimuliert die krankhaft aktive Neuronenpopulation 31 im Gehirn und/oder Rückenmark 30 mit den elektrischen und/oder optischen Reizen 22 entweder direkt oder aber die Reize 22 werden über das Nervensystem an die krankhaft aktive Neuronenpopulation 31 weitergeleitet. Die Reize 22 sind so ausgestaltet, dass die zeitversetzte (oder phasenverschobene) Stimulation mit mindestens zwei Stimulationselementen eine Desynchronisation der krankhaft synchronen Aktivität der Neuronenpopulation 31 bewirkt. Eine durch die Stimulation bewirkte Senkung der Koinzidenzrate der Neuronen kann zu einer Senkung der synaptischen Gewichte und somit zu einem Verlernen der Tendenz zur Produktion krankhaft synchroner Aktivität führen.

Die bei der CR-Stimulation verabreichten Reize 22 bewirken in der Neuronenpopulation 30 ein Zurücksetzen, einen sogenannten Reset, der Phase der neuronalen Aktivität der stimulierten Neuronen. Durch das Zurücksetzen wird die Phase der stimulierten Neuronen unabhängig von dem aktuellen Phasenwert auf einen oder nahe zu einem bestimmten Phasenwert, z. B. 0°, gesetzt (in der Praxis ist es nicht möglich, einen bestimmten Phasenwert exakt einzustellen, dies ist für eine erfolgreiche CR-Stimulation aber auch nicht erforderlich). Somit wird die Phase der neuronalen Aktivität der krankhaften Neuronenpopulation 31 mittels einer gezielten Stimulation kontrolliert. Da die krankhafte Neuronenpopulation 31 über die Stimulationselemente 12 bis 15 an unterschiedlichen Stellen stimuliert wird, können die jeweiligen Phasen der neuronalen Aktivität der in Fig. 1 dargestellten Subpopulationen 32 bis 35 der krankhaften Neuronenpopulation 31 zu unterschiedlichen Zeitpunkten zurückgesetzt werden, indem die Reize 22 von den Stimulationselementen 12 bis 15 zeitversetzt (oder phasenverschoben) appliziert werden. Im Ergebnis wird dadurch die krankhafte Neuronenpopulation 31, deren Neuronen zuvor synchron und mit gleicher Frequenz und Phase aktiv waren, in mehrere Subpopulationen mit unterschiedlichen Phasen aufgespalten. Beispielsweise stimuliert das Stimulationselement 12 die Subpopulation 32, das Stimulationselement 13 stimuliert die Subpopulation 33, das Stimulationselement 14 stimuliert die Subpopulation 34 und das Stimulationselement 15 stimuliert die Subpopulation 35. Innerhalb jeder der Subpopulationen 32 bis 35 sind die Neuronen nach dem Zurücksetzen der Phase weiterhin synchron und feuern auch weiterhin mit derselben pathologischen Frequenz, aber jede der Subpopulationen 32 bis 35 weist bezüglich ihrer neuronalen Aktivität die Phase auf, die ihr durch den von dem jeweiligen Stimulationselement 32 bis 35 generierten Reiz 22 aufgezwungen wurde. Dies bedeutet, dass die neuronalen Aktivitäten der einzelnen Subpopulationen 32 bis 35 nach dem Zurücksetzen ihrer Phasen weiterhin einen in etwa sinusförmigen Verlauf mit derselben pathologischen Frequenz haben, aber unterschiedliche Phasen.

Wie vorstehend beschrieben stimulieren die Stimulationselemente 12 bis 15 mit den Reizen 22 unterschiedliche Subpopulationen. Es muss sich dabei aber nicht notwendigerweise um disjunkte, d. h. vollständig voneinander getrennte Subpopulationen handeln. Die von den Stimulationselementen 12 bis 15 stimulierten Subpopulationen können einander auch überlappen.

Bedingt durch die krankhafte Interaktion zwischen den Neuronen ist der durch die Stimulation erzeugte Zustand mit mindestens zwei Subpopulationen instabil, und die gesamte Neuronenpopulation 31 nähert sich schnell einem Zustand kompletter Desynchronisation, in welchem die Neuronen unkorreliert feuern. Der gewünschte Zustand, d. h. die komplette Desynchronisation, ist somit nach der zeitversetzten (oder phasenverschobenen) Applikation der phasenrücksetzenden Reize 22 nicht sofort vorhanden, sondern stellt sich meist innerhalb weniger Perioden oder gar in weniger als einer Periode der pathologischen Frequenz ein.

Eine Theorie zur Erklärung des Stimulationserfolgs basiert darauf, dass die letztlich gewünschte Desynchronisation durch die krankhaft gesteigerte Interaktion zwischen den Neuronen erst ermöglicht wird. Hierbei wird ein Selbstorganisationsprozess ausgenutzt, der für die krankhafte Synchronisation verantwortlich ist. Derselbe bewirkt, dass auf eine Aufteilung einer Gesamtpopulation 31 in Subpopulationen 32 bis 35 mit unterschiedlichen Phasen eine Desynchronisation folgt. Im Gegensatz dazu würde ohne krankhaft gesteigerte Interaktion der Neuronen keine Desynchronisation erfolgen.

Darüber hinaus kann durch die CR-Stimulation eine Neuorganisation der Konnektivität der gestörten neuronalen Netzwerke erzielt werden, so dass lang anhaltende therapeutische Effekte bewirkt werden können. Der erzielte synaptische Umbau ist von großer Bedeutung für die wirksame Behandlung neurologischer oder psychiatrischer Erkrankungen.

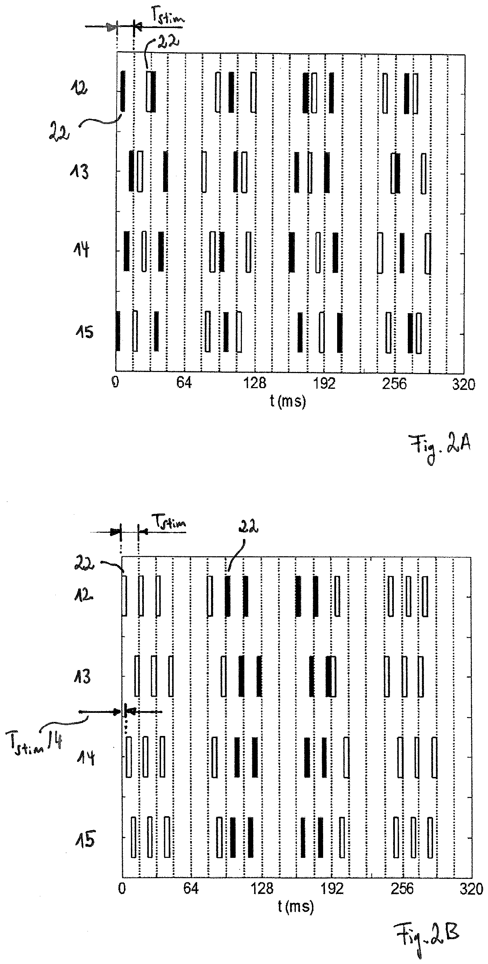

Fig. 2A zeigt eine CR-Stimulation, bei der die vier Stimulationselemente 12 bis 15 repetitiv Sequenzen von Reizen 22 erzeugen. In Fig. 2A sind untereinander die von den Stimulationselementen 12 bis 15 erzeugten Reize 22 gegen die Zeit t aufgetragen. Die Sequenzen werden in einem vorgegebenen Zeitraster, das aus aufeinanderfolgenden Zyklen besteht, erzeugt. Die einzelnen Zyklen sind in Fig. 2A durch gestrichelte Linien voneinander abgegrenzt. Jeder Zyklus weist die Länge $T_{stim}$ auf. In jedem Zyklus, in dem eine Stimulation erfolgt, erzeugen die Stimulationselemente 12 bis 15 zusammen genau eine Sequenz von Reizen 22 und jedes Stimulationselement 12 bis 15 erzeugt genau einen Reiz 22, d. h., jede Sequenz besteht in dem vorliegenden Beispiel aus einer Abfolge von vier zeitversetzten Reizen 22, die insbesondere von jeweils unterschiedlichen Stimulationselementen 12 bis 15 generiert werden, wobei sich der Zeitversatz insbesondere auf die Anfangszeitpunkte der Reize 22 beziehen kann. Zu Beginn jedes Zyklus wird in dem vorliegenden Beispiel die Reihenfolge, in welcher die Stimulationselemente 12 bis 15 die Reize 22 erzeugen, variiert. Eine unterschiedliche Füllung der in Fig. 2A gezeigten Balken, welche die Reize 22 symbolisieren, zeigt eine Variation der Reihenfolge an. Beispielsweise erzeugen die Stimulationselemente 12 bis 15 in dem ersten in Fig. 2A gezeigten Zyklus die Reize 22 in der Reihenfolge 15-12-14-13. Im zweiten Zyklus lautet die Reihenfolge 15-13-14-12 und im dritten Zyklus lautet die Reihenfolge 12-15-14-13.

Ferner werden bei der in Fig. 2A gezeigten beispielhaften Stimulationsform stets in drei aufeinanderfolgenden Zyklen Reize 22 appliziert und danach wird für zwei Zyklen eine Pause eingehalten, in der keine Reize 22 generiert werden. Dieses Muster wird periodisch wiederholt.

Fig. 2B zeigt eine Weiterentwicklung der CR-Stimulation von Fig. 2A. Der wesentliche Unterschied zur Stimulation nach Fig. 2A besteht darin, dass bei der in Fig. 2B dargestellten CR-Stimulation die Sequenzen nur sehr langsam variiert werden. Insbesondere ist vorgesehen, dass die Reihenfolge, in welcher die Stimulationselemente 12 bis 15 innerhalb einer Sequenz die Reize 22 erzeugen, für mindestens 20 nacheinander generierte Sequenzen konstant gehalten und erst danach variiert wird. Eine CR-Stimulation mit derart langsam variierenden Sequenzen ist gegenüber der in Fig. 2A gezeigten CR-Stimulation erheblich überlegen, da ihr gewünschter, d. h. therapeutischer Stimulationseffekt (i) stärker ausgeprägt ist, (ii) von Stimulationsepoche zu Stimulationsepoche deutlich weniger variiert und (iii) deutlich robuster gegenüber Schwankungen der Reizintensität, gegenüber Schwankungen charakteristischer Kenngrößen des Körpers bzw. Nervensystems sowie insbesondere gegenüber Variationen der Anfangswerte ist.

Allgemein bekannt ist, dass beim Lernen die Wiederholung des zu lernenden Inhalts eine wichtige Rolle spielt. Die Erfindung nutzt den überraschenden Zusammenhang, dass auch beim Verlernen das Wiederholen von überaus großer Bedeutung ist. D. h., um krankhaft synchrone synaptische Vernetzungen und damit krankhaft synchrone neuronale Aktivität erheblich besser zu verlernen, sollten die Sequenzen der CR-Stimulation nur langsam variiert werden, so dass jede einzelne Sequenz häufig genug wiederholt wird.

Wie oben beschrieben kann vorgesehen sein, dass die Sequenzen für mindestens 20 nacheinander generierte Sequenzen gleich bleiben und erst danach geändert werden. Es ist weiterhin denkbar, die Wiederholung derselben Sequenz zu erhöhen und die Reihenfolge, in welcher die Stimulationselemente 12 bis 15 pro Zyklus die Reize 22 erzeugen, für mindestens 25 oder mindestens 30 nacheinander generierte Sequenzen konstant zu halten. Es sei an dieser Stelle noch darauf hingewiesen, dass in Fig. 2B aus Gründen der Veranschaulichung die Sequenzen bereits nach weniger als 20 Sequenzen variiert werden. Dies ist jedoch lediglich als eine vereinfachte Darstellung einer im Vergleich zu Fig. 2A langsamen Sequenzvariation zu verstehen.

Gemäß einer Ausgestaltung wird bei der in Fig. 2B gezeigten CR-Stimulation nur die Reihenfolge, in welcher die Stimulationselemente 12 bis 15 pro Sequenz die Reize 22 erzeugen, variiert. Alle übrigen Stimulationsparameter können während der CR-Stimulation konstant bleiben.

Die Variation der Sequenzen kann z. B. stochastisch oder deterministisch oder gemischt stochastisch-deterministisch erfolgen.

Genauso wie in Fig. 2A können auch bei der CR-Stimulation nach Fig. 2B Zyklen vorgesehen sein, in denen Stimulationspausen eingehalten werden. So können während n aufeinanderfolgenden Zyklen Reize 22 erzeugt werden und während der darauffolgenden m Zyklen keine Reize 22 erzeugt werden, die dazu ausgelegt sind, die krankhaft synchrone und oszillatorische neuronale Aktivität zu unterdrücken, wobei n und m nicht-negative ganze Zahlen sind. Es ist jedoch denkbar, dass andere Reize, die nicht dazu ausgelegt sind, die krankhaft synchrone und oszillatorische neuronale Aktivität zu unterdrücken, während der Stimulationspausen insbesondere mit der Stimulationseinheit 11 appliziert werden. Weiterhin kann vorgesehen sein, dass die Stimulationseinheit 11 während der Stimulationspausen keinerlei Reize erzeugt. Das Muster aus n Zyklen mit Stimulation und m Zyklen ohne Stimulation kann periodisch fortgesetzt werden.

Sofern vorgesehen ist, die Sequenzen nach einer vorgegebenen Anzahl i von Sequenzen zu variieren ($i \geq 20$),

werden gemäß einer Ausgestaltung die Zyklen ohne Stimulation nicht mitgezählt, d. h., es findet bei dieser Ausgestaltung eine Variation der Reihenfolge, in der die Stimulationselemente 12 bis 15 die Reize 22 generieren, erst dann statt, wenn tatsächlich in i Zyklen jeweils eine Sequenz von Reizen 22 appliziert wurde. Die Anzahl i, nach der jeweils die Sequenz variiert wird, kann z. B. gemäß stochastischen oder deterministischen oder gemischt stochastischdeterministischen Regeln bestimmt werden.

Weiterhin kann die Variation der Sequenzen mit einem konstanten Rhythmus erfolgen, d. h., eine Variation findet beispielsweise stets nach i Zyklen statt.

Jedes der vier Stimulationselemente 12 bis 15 stimuliert eine jeweilige der in Fig. 1 dargestellten Subpopulationen 32 bis 34 der krankhaften Neuronenpopulation 31. Während der mindestens 20 Zyklen, in denen die Sequenzen konstant sind, wird von jedem der Stimulationselemente 12 bis 15 der Reiz 22 periodisch mit der Periode $T_{stim}$ appliziert. Die Reize 22 bewirken eine Phasenrücksetzung der neuronalen Aktivität der jeweils stimulierten Subpopulation. Ferner beträgt die zeitliche Verzögerung zwischen innerhalb einer Sequenz zeitlich direkt aufeinanderfolgenden, von unterschiedlichen Stimulationselementen erzeugten Reizen 22 $T_{stim}/4$, da in dem vorliegenden Ausführungsbeispiel vier Stimulationselemente 12 bis 15 für die CR-Stimulation eingesetzt werden. Für den allgemeinen Fall von P für die Stimulation verwendeten Stimulationselementen würde die zeitliche Verzögerung zwischen innerhalb einer Sequenz zeitlich direkt aufeinanderfolgenden, von unterschiedlichen Stimulationselementen erzeugten Reizen 22 $T_{stim}/P$ betragen (von diesem Wert kann auch um z. B. bis zu $\pm$ 5%, $\pm$ 10% oder $\pm$ 20% abgewichen werden). Die zeitliche Verzögerung $T_{stim}/P$ kann sich auf die Anfangszeitpunkte der Reize 22 beziehen. Die von unterschiedlichen Stimulationselementen erzeugten Reize 22 können bis auf die unterschiedlichen Startzeitpunkte identisch sein.

Die Periode $T_{stim}$, die zum einen die Dauer eines Zyklus und zum anderen die Periode angibt, mit der gleich bleibende Sequenzen sowie die von einem jeweiligen Stimulationselement 12 bis 15 generierten Reize 22 wiederholt werden, kann nahe bei der mittleren Periode der pathologischen Oszillation der Neuronenpopulation 31 mit der krankhaft synchronen und oszillatorischen neuronalen Aktivität liegen bzw. um bis zu $\pm$ 5%, $\pm$ 10% oder $\pm$ 20% von der mittleren Periode abweichen. Typischerweise liegt die Frequenz $f_{stim} = 1/T_{stim}$ im Bereich von 1 bis 30 Hz. Die Periode der pathologischen Oszillation der zu stimulierenden Neuronenpopulation 31 kann, beispielsweise mittels EEG, gemessen werden. Es ist aber auch möglich, für die Periode der pathologischen Oszillation Literatur- oder Erfahrungswerte, die sich auf die jeweilige, zu behandelnde Krankheit beziehen, zu verwenden.

Die phasenrücksetzenden Reize 22 können beispielsweise Einzelreize oder auch zusammengesetzte Reize sein. Beispielsweise kann jeder Reiz 22 aus einem Pulszug mit 1 bis 100, insbesondere 2 bis 10 Einzelpulsen bestehen. Innerhalb eines Pulszugs werden die Einzelpulse ohne Unterbrechung mit einer Frequenz im Bereich von 50 bis 500 Hz, insbesondere im Bereich von 100 bis 150 Hz, wiederholt.

Die Stimulationseinheit 11 kann grundsätzlich eine beliebige Anzahl L von Stimulationselementen enthalten ($L \geq 2$), jedoch müssen bei einer Stimulation nicht zwingend alle L Stimulationselemente verwendet werden, es kann beispielsweise auch nur eine Auswahl von P der L Stimulationselemente die Reize 22 erzeugen ($2 \leq P \leq L$). Bei P Stimulationselementen ergeben sich P! mögliche unterschiedliche Sequenzen, wobei bei jeder dieser Sequenzen jedes der P Stimulationselemente genau einen Reiz 22 erzeugt. Es ist denkbar, alle P! möglichen Sequenzen für die Stimulation heranzuziehen oder auch aus der Menge der P! möglichen Sequenzen eine Untermenge für die Stimulation auszuwählen. Diese Untermenge kann auch in der Zeit gemäß stochastisch oder deterministisch oder gemischt stochastisch-deterministisch Regeln variieren. Die Abfolge der Sequenzen kann zufällig sein oder vor Beginn oder auch während der Stimulation festgelegt werden.

Die in Fig. 1 dargestellte Vorrichtung 1 zur Stimulation von Neuronen mit einer krankhaft synchronen und oszillatorischen neuronalen Aktivität führt eine sogenannte "open loop"-Stimulation durch, d. h., eine Stimulation ohne Sensoren, die zur Rückmeldung und/oder Steuerung der Stimulation verwendet werden.

Fig. 3 zeigt schematisch eine Vorrichtung 2 zur Stimulation von Neuronen mit einer krankhaft synchronen und oszillatorischen neuronalen Aktivität, mit der sich eine "closed loop"-Stimulation durchführen lässt. Die Vorrichtung 2 ist eine Weiterbildung der in Fig. 1 dargestellten Vorrichtung 1 und enthält genauso wie die Vorrichtung 1 eine Steuereinheit 10 und eine implantierbare Stimulationseinheit 11, welche die gleichen Funktionen wie die oben beschriebenen Steuer- und Stimulationseinheiten 10, 11 der Vorrichtung 1 aufweisen.

Darüber hinaus umfasst die Vorrichtung 2 eine Messeinheit 16. Der durch die Reize 22 erzielte Stimulationseffekt wird mit Hilfe der Messeinheit 16 überwacht. Die Messeinheit 16 nimmt ein oder mehrere am Patienten gemessene Messsignale 23 auf, wandelt diese gegebenenfalls in elektrische Signale 24 um und führt sie der Steuereinheit 10 zu. Insbesondere kann mittels der Messeinheit 16 die neuronale Aktivität in dem stimulierten Zielgebiet oder einem mit dem Zielgebiet verbundenen Gebiet gemessen werden, wobei die neuronale Aktivität dieses Gebiets mit der neuronalen Aktivität des Zielgebiets hinreichend eng korreliert. Mittels der Messeinheit 16 kann auch eine nicht-neuronale, z. B. muskuläre Aktivität oder die Aktivierung des autonomen Nervensystems, gemessen werden, sofern diese mit der neuronalen Aktivität des Zielgebiets hinreichend eng korreliert sind.

Die Messeinheit 16 enthält ein oder mehrere Sensoren, die es insbesondere ermöglichen, eine Ab- bzw. Zunahme der Amplitude der pathologischen oszillatorischen Aktivität nachzuweisen.

Als Sensoren können nicht-invasive Sensoren eingesetzt werden, z. B. chronisch oder intermittent genutzte Elektroenzephalographie (EEG)- oder Elektromyographie (EMG)-Elektroden oder Magnetenzephalographie (MEG)-Sensoren. Die neuronale Aktivität kann auch durch Detektion charakteristischer Bewegungsmuster wie Tremor, Akinese oder epileptische Anfälle mit Hilfe eines Akzelerometers oder Gyroskops oder indirekt durch Messung der Aktivierung des autonomen Nervensystems mittels Messung des Hautleitwiderstands ermittelt werden. Es können auch Befindlichkeitswerte, die vom Patienten in portable Geräte, z. B. Smartphones, eingegeben werden, zur Kontrolle des Stimulationserfolgs verwandt werden.

Alternativ können die Sensoren in den Körper des Patienten implantiert sein. Als invasive Sensoren können beispielsweise epikortikale Elektroden, Tiefenhirnelektroden zur Messung von z. B. lokalen Feldpotentialen, sub- oder epidurale Hirnelektroden, subkutane EEG-Elektroden und sub- oder epidurale Rückenmarkselektroden dienen. Die Tiefenelektroden zur Messung der lokalen Feldpotentiale können auch baulich vereint oder sogar identisch mit den für die Stimulation verwendeten Tiefenelektroden sein.

Die Steuereinheit 10 verarbeitet die Signale 24, z. B. können die Signale 24 verstärkt und/oder gefiltert werden, und analysiert die verarbeiteten Signale 24. Die Steuereinheit 10 überprüft anhand der in Reaktion auf die Applikation der Reize 22 aufgenommenen Messsignale 23 den Stimulationserfolg.

Gemäß einer Ausgestaltung werden die von der Stimulationseinheit 11 erzeugten Sequenzen mit konstantem Rhythmus variiert, d. h., die Reihenfolge, in welcher die Stimulationselemente 12 bis 15 pro Sequenz die Reize erzeugen, wird für eine vorgegebene Zahl von Zyklen (mindestens 20) konstant gehalten und danach wird die Reihenfolge variiert. Anschließend wird die Reihenfolge für die vorgegebene Zahl von Zyklen wieder konstant gehalten und danach variiert. Dieses Muster wird entsprechend fortgesetzt. Bei dieser Ausgestaltung bleibt der Rhythmus, mit dem die Sequenzen variiert werden, konstant und wird insbesondere nicht an die von der Steuereinheit 10 verarbeiteten Messsignale 23 angepasst, jedoch können andere Stimulationsparameter, wie z. B. die Amplitude der als Reize 22 applizierten Pulszügen, in Abhängigkeit von den Messsignalen 23 eingestellt werden.

Die vorstehende Ausgestaltung kann weitergebildet werden, indem die Stimulationsparameter in Abhängigkeit von den verarbeiteten Messsignalen 23 eingestellt werden. Die Steuereinheit 10 überprüft anhand der in Reaktion auf die Applikation der Reize 22 aufgenommenen Messsignale 23 den Stimulationserfolg und stellt die Stimulationsparameter, insbesondere den Rhythmus, mit der die Stimulationssequenzen variiert werden, in Abhängigkeit vom Stimulationserfolg ein.

Der Stimulationserfolg kann insbesondere mittels eines Schwellwertvergleichs überprüft werden. Je nachdem welche Signale zur Ermittlung des Stimulationserfolgs herangezogen werden, ergeben sich unterschiedliche Schwellwertvergleiche. Wird z. B. die krankhafte neuronale Synchronisation über die Sensoren der Messeinheit 16, z. B. EEG-Elektroden oder Tiefelektroden (als LFP-Signal), gemessen, reicht erfahrungsgemäß die Absenkung der Synchronisation um z. B. mindestens 20 % im Vergleich zur Situation ohne Stimulation, um einen ausreichenden Stimulationserfolg festzustellen. Gemäß einer Ausgestaltung kann ein nicht ausreichender Stimulationserfolg festgestellt werden, wenn sich die krankhafte neuronale Synchronisation durch die Applikation der Reize 22 nicht um mindestens einen vorgegebenen Wert verringert. Falls Symptome des Patienten zur Ermittlung des Stimulationserfolgs herangezogen werden, hängt es von der Art der verwandten klinischen Parameter ab, welche Abnahme als klinisch relevante Besserung anzusehen ist. Derartige Abnahmewerte (z. B. im Sinne der sog. minimalen klinisch wahrnehmbaren Verbesserung) sind dem Fachmann bekannt.

Wenn die CR-Stimulation gemäß Schwellwertvergleich nicht hinreichend effektiv ist, d. h., ein krankheitsspezifischer Marker im Vergleich zum Ausgleichszustand bzw. zu einem Start-/Ausgangswert durch die CR-Stimulation nicht um einen vorgegebenen Schwellwert abnimmt, wird die Anzahl der Wiederholungen derselben Sequenz verlängert. Ist die Stimulation hingegen gemäß Schwellwertkriterium erfolgreich, wird die Anzahl der Wiederholungen derselben Sequenz verkürzt. Im einfachsten Fall kann dies ein binäres Schalten zwischen zwei Werten der Anzahl der Wiederholungen derselben Sequenz sein: z. B. 25 Wiederholungen bei erfolgreicher Stimulation, hingegen z. B. 100 Wiederholungen bei nicht erfolgreicher Stimulation. Die bedarfsgesteuerte Anzahl der Wiederholungen derselben Sequenz kann aber auch in kleineren Schritten variiert/ parametrisiert werden.

Die einzelnen Komponenten der Vorrichtungen 1 und 2, insbesondere die Steuereinheit 10, die Stimulationseinheit 11 und/oder die Messeinheit 16, können baulich voneinander getrennt sein. Die Vorrichtungen 1 und 2 können daher auch als Systeme aufgefasst werden. Zur Durchführung ihrer Aufgaben kann die Steuereinheit 10 einen Prozessor, z. B. einen Mikrocontroller, enthalten. Die hierin beschriebenen Stimulationsverfahren können als Software-Code in einem der Steuereinheit 10 zugeordneten Speicher abgelegt sein.

Die Stimulationseinheit 11 kann z. B. ein Hirnschrittmacher sein und weist in diesem Fall eine oder mehrere implantierbare Elektroden, z. B. Tiefenelektroden, sowie gegebenenfalls dazwischen geschaltete Verbindungskabel auf. Die Elektroden der Stimulationseinheit 11 bestehen typischerweise aus einem isolierten Elektrodenschaft und einer Mehrzahl von Stimulationskontaktflächen, die in den Elektrodenschaft eingebracht worden sind.

Fig. 4 zeigt schematisch eine Vorrichtung 40 zur invasiven elektrischen Stimulation von Neuronen mit einer krankhaft synchronen und oszillatorischen neuronalen Aktivität gemäß einer Ausführungsform der Erfindung. Die Vorrichtung 40 umfasst zwei Elektroden 41, 42, die in das Gehirn des Patienten implantiert sind und über Kabel 43 mit einem Konnektor 44 verbunden sind. Der Konnektor 44 wiederum ist über ein Kabel 45 mit einer Steuereinheit 46 verbunden. Die Vorrichtung 40 kann die Funktionen der oben beschriebenen Vorrichtungen 1 und 2 aufweisen.

Implantierbare Stimulationseinheiten für die optische Stimulation von neuronalem Gewebe sind bekannt. Beispielsweise kann eine Lichtquelle, wie z. B. ein Laser, eine Laserdiode oder eine LED, einen Lichtstrahl erzeugen, der mit Hilfe einer Lichteinkopplung auf die Eingänge eines aus mehreren Lichtleitern bestehenden Faserbündels verteilt wird. Eine Steuereinheit gibt dabei z. B. vor, zu welchem Zeitpunkt ein einzelner Lichtpuls oder ein Zug von Lichtpulsen in welche Faser des Faserbündels eingekoppelt wird. Die Auskopplungspunkte der einzelnen Fasern des Fasernbündels, d. h. die Enden der Fasern, liegen an verschiedenen Stellen im Zielgebiet im Gehirn oder Rückenmark des Patienten. Das Licht stimuliert somit unterschiedliche Orte des Zielgebiets in einer durch die Steuereinheit vorgegebenen zeitlichen Abfolge. Es können allerdings auch andere implantierbare Stimulationseinheiten verwendet werden, die sich zur direkten optischen Stimulation von neuronalem Gewebe eignen.

Wie oben beschrieben bewirken die Reize 22 bei der CR-Stimulation ein Zurücksetzen, einen sogenannten Reset, der Phase der neuronalen Aktivität der stimulierten Neuronen. Mit Hilfe der von der Messeinheit 16 aufgenommenen Messsignale 23 kann die Phasenrücksetzung der einzelnen Reize 22 überprüft werden. Eine derartige Untersuchung kann vor der eigentlichen therapeutischen Neurostimulation vorgenommen werden.

Dazu wird über einen Sensor der Messeinheit 16 ein Signal gemessen, welches die Aktivität der über den j-ten Stimulationskanal stimulierten Subpopulation hinreichend repräsentiert. Ein Stimulationskanal kann repräsentiert sein durch ein Stimulationselement, z. B. eines der Stimulationselemente 12 bis 15, welches eine bestimmte Subpopulation stimuliert. Man erhält das vorstehende Signal entweder direkt von der Subpopulation über eine nicht-invasive Messung, z. B. über EEG- oder MEG-Elektroden, oder eine invasive Messung, z. B. über implantierte Elektroden, als Oberflächen-EEG oder als lokales Feldpotential über Tiefenelektroden. Das Signal kann auch indirekt über die Messung einer mit der Aktivität der stimulierten Subpopulation korrelierten Größe ermittelt werden. Hierzu eignen sich z. B. EEG-/MEG-/LFP-Signale der neuronalen Aktivität einer mit dieser Subpopulation eng gekoppelten anderen Neuronenpopulation oder zugehörige Elektromyographie-, Akzelerometer- oder Gyroskop-Signale.

Da neuronale Signale typischerweise rhythmische Aktivität in unterschiedlichen Frequenzbändern enthalten, ist es in solchen Fällen vorteilhaft, z. B. mittels Bandpassfilterung oder wavelet-Analyse oder empirical mode decomposition das Signal $x_j(t)$, welches die pathologische oszillatorische Aktivität der vom j-ten Stimulationskanal stimulierten Subpopulation repräsentiert, zu ermitteln.

Ein nur wenig aufwändiges Vorgehen, um eine Phasenrücksetzung zu überprüfen, besteht darin, die gemittelte Reizantwort zu bestimmen. Hierzu wird zu den Zeiten $\tau_1$, $\tau_2$, ..., $\tau_1$ ein Reiz mit identischen Reizparametern appliziert. Die Abstände zwischen den einzelnen Reizen $\tau_{k+1}$ - $\tau_k$ sollten hinreichend groß und randomisiert, also nicht konstant sein, um Einschwingvorgänge zu vermeiden (vgl. P. A. Tass: Transmission of stimulus-locked responses in two coupled phase oscillators. Phys. Rev. E 69, 051909-1-24 (2004)). Typischerweise sollten die Abstände $\tau_{k+1}$ - $\tau_k$ im Bereich von mindestens dem Zehnfachen, besser dem Hundertfachen der mittleren Periode des pathologischen Oszillation liegen. Die über alle 1 Test-Reize gemittelte Reizantwort wird gemäß folgender Gleichung berechnet:

$$\overline{x}_j(t) = \frac{1}{l}\sum_{k=1}^{l} x_j(\tau_k + t) \tag{1}$$

Sofern die Abstände $\tau_{k+1}$ - $\tau_k$ zwischen den einzelnen Reizen hinreichend groß sind, erhält man im prä-Stimulus-Bereich, d. h. im Bereich vor der Applikation eines jeweiligen Reizes, keine gemittelte Reizantwort (vgl. P. A. Tass: Transmission of stimulus-locked responses in two coupled phase oscillators. Phys. Rev. E 69, 051909-1-24 (2004)). Eine Phasenrücksetzung kann festgestellt werden, wenn eine gemittelte Reizantwort detektiert werden kann, d. h., wenn sich im post-Stimulus-Bereich, d. h. im Bereich für t > 0, wobei t = 0 den Anfangszeitpunkt des jeweiligen Reizes darstellt, eine von Null verschiedene Reizantwort findet. Dies kann durch visuelle Inspektion ermittelt werden. Man kann dies auch von der Vorrichtung 2, insbesondere der Steuereinheit 10, durchführen lassen, indem man die prä-Stimulus-Verteilung von $\overline{x}_j(t)$ oder $|\overline{x}_j(t)|$ betrachtet und einen charakteristischen Schwellwert, z. B. die 99te Perzentile der prä-Stimulus-Verteilung von $|\overline{x}_j(t)|$ oder schlicht deren Maximum bestimmt. Wenn nun z. B. der Betrag der post-Stimulus-Antwort prinzipiell oder für eine vorgegebene Mindestdauer, z. B. 20 ms, diesen charakteristischen Schwellenwert übersteigt, liegt eine von Null verschiedene gemittelte Antwort vor. In diesem Fall kann eine Phasenrücksetzung vorliegen. D. h., die Reizstärke müsste so lange erhöht werden, bis die post-Stimulus-Antwort sich von einer Nulllinie unterscheidet. Neben dem hier vorgestellten einfachen, aber in der Praxis bewährten Verfahren können auch andere, dem Fachmann bekannte statistische Tests zur Signalanalyse herangezogen werden.

Eine genauere, aber aufwändigere Variante zur Untersuchung, ob die Reize eine Phasenrücksetzung bewirken, bietet die Analyse der Phase. Hierzu wird die Phase $\psi_j(t)$ von $x_j(t)$ bestimmt. Dies erfolgt mittels Hilbert-Transformation aus dem mittels Bandpassfilterung bzw. empirical mode decomposition bestimmten Signal, welches die pathologische oszillatorische Aktivität repräsentiert. Die empirical mode decomposition ermöglicht im Vergleich zur Bandpassfilterung eine parameterunabhängige Bestimmung physiologisch relevanter Moden in verschiedenen Frequenzbereichen (vgl. N. E. Huang et al.: The empirical mode decomposition and the Hilbert spectrum for nonlinear and non-stationary time series analysis. Proc. R. Soc. A: Math. Phys. Eng. Sci. 454:903-995 (1998)). Die Kombination von empirical mode decomposition mit nachfolgender Hilbert-Transformation wird als Hilbert-Huang-Transformation bezeichnet (vgl. N. E. Huang et al.: A confidence limit for the empirical mode decomposition and Hilbert spectral analysis, Proceedings of the Royal Society of London Series A, 459, 2317-2345 (2003)). Die Phase $\psi_j(t)$ kann auch mittels Wavelet-Analyse bestimmt werden.

Eine Phasenrücksetzung liegt vor, wenn die Phase $\psi_j(t)$ durch einen Reiz (mit Reiz-Beginn bei t = 0) nach einer bestimmten Zeit auf einen Vorzugswert gesetzt wird. D. h., $\{\psi_j(\tau_k + t)\}_{k=1,\ldots,l}$, die von den l Reizantworten gewonnene Verteilung der Werte der Phase $\psi_j(t)$ hat zur Zeit t (relativ zum Burst-Beginn bei t = 0) einen Häufungswert. Dem Fachmann sind unterschiedliche Methoden bekannt, mit denen sich nachweisen lässt, dass eine Verteilung einen Häufungswert (also einen Peak) hat. Eine gebräuchliche Methode ist die Bestimmung des Phasenrücksetzungsindex $\rho(t)$ mittels zirkulärem Mittelwert:

$$\rho(t) = \left| \frac{1}{l} \sum_{k=1}^{l} \exp\left[ i \psi_j(\tau_k + t) \right] \right| \qquad (2)$$

Eine Phasenrücksetzung liegt vor, wenn $\rho(t)$ z. B. das Maximum oder die 99te Perzentile der prä-Stimulus-Verteilung von $\rho(t)$ (an einem Zeitpunkt oder innerhalb eines kleinen Zeitfensters von z. B. 20 ms Breite) überschreitet.

In der Praxis hat sich die Analyse mit den gemittelten Antworten $\bar{x}_j(t)$ als ausreichend bewährt.

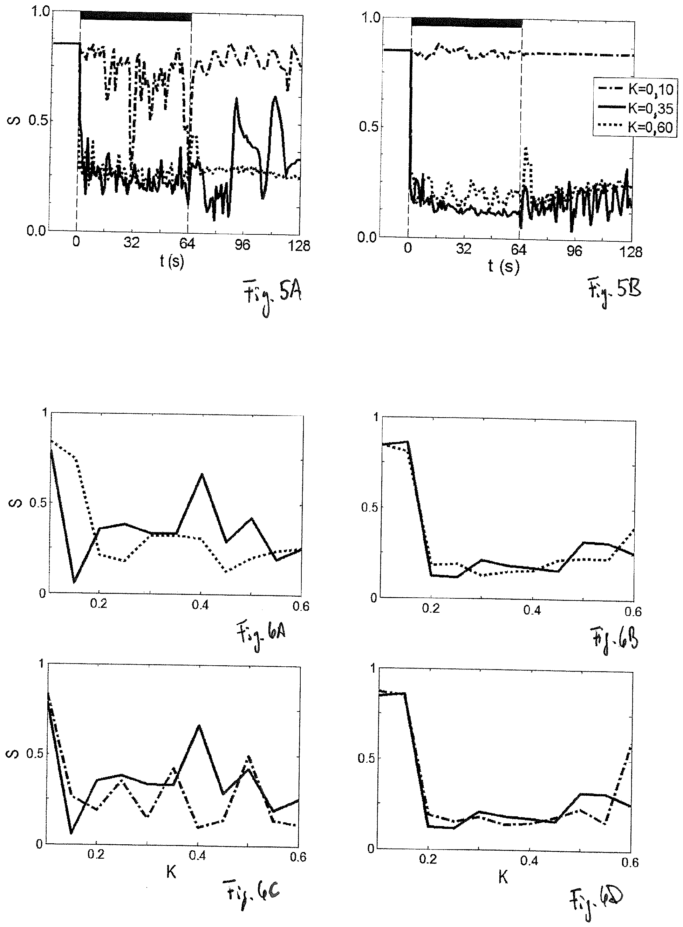

In den Fig. 5 bis 7 werden die mit der hierin beschriebenen Erfindung erzielbaren Effekte anhand von Simulationsergebnissen veranschaulicht. Der Simulation liegt ein Netzwerk von 200 Neuronen zugrunde, wobei alle Neuronen untereinander eine starke anregende kurzreichweitige Kopplung und eine schwache inhibitorische langreichweitige Kopplung aufweisen. Die synaptischen Kopplungsstärken in dem Netzwerk können sich gemäß STDP (Spike Timing-Dependent Plasticity)-Regeln ändern. Ein anfänglich stark gekoppeltes Netzwerk produziert hoch synchrone neuronale Aktivität.

Die Simulation basiert auf folgenden Randbedingungen. Die CR-Stimulation startet bei t = 0 s und endet bei t = 64 s. Jeder Zyklus dauert 16 ms. Ein Muster aus 3 Zyklen mit Stimulation und 2 Zyklen ohne Stimulation wird periodisch wiederholt. Die Aktivität des Netzwerks wird bis t = 128 s untersucht, d. h., bis 64 s nach Ende der Stimulation. Der Synchronisationsgrad S kann im Bereich von 0 (für eine komplette Desynchronisation) bis 1 (für eine vollständige Phasensynchronisation) liegen.

In den Fig. 5A und 5B ist der Synchronisationsgrad S der simulierten Neuronenpopulation mit einer krankhaft synchronen und oszillatorischen neuronalen Aktivität vor, während und nach einer CR-Stimulation dargestellt. Die in den beiden Darstellungen oben eingezeichneten horizontalen Balken geben den Zeitraum an, in dem die CR-Stimulation angewandt wird. Bei der in Fig. 5A dargestellten Simulation wurden die Sequenzen zu Beginn jedes Zyklus variiert, während die Sequenzen bei der in Fig. 5B dargestellten Simulation erst nach jeweils 100 Zyklen variiert wurden. Der Synchronisationsgrad S wurde nach jeder Millisekunde berechnet. In den Fig. 5A und 5B ist jeweils die 50. Perzentile der Werte für den Synchronisationsgrad S für drei verschiedene Stimulationsstärken K dargestellt.

Als Ergebnis lässt sich den Fig. 5A und 5B entnehmen, dass bis auf die CR-Stimulation mit einer Stimulationsstärke K von 0,10 die in Fig. 5B dargestellte langsam variierende CR-Stimulation einen größeren und auch länger anhaltenden Stimulationserfolg zeigt.

Neben der Stimulationsstärke K haben auch die Reihenfolge der Sequenzen und die Anfangsbedingungen des Netzwerks Einfluss auf den Stimulationserfolg. Dies ist in den Fig. 6A bis 6D gezeigt, in denen der Synchronisationsgrad S gegen die Stimulationsstärke K aufgetragen ist. Den in den Fig. 6A und 6C dargestellten Simulationen liegt eine Variation der Sequenzen in jedem Zyklus zugrunde, wohingegen bei den in den Fig. 6B und 6D gezeigten Simulationen die Sequenzen erst nach 100 Zyklen variiert wurden.

In den Fig. 6A und 6B ist jeweils die 50. Perzentile der Werte für den Synchronisationsgrad S für die letzten 16 Sekunden der Stimulation als eine Funktion der Stimulationsstärke K für zwei verschiedene Reihenfolgen der Sequenzen dargestellt. Die durchgezogenen Linien zeigen die Simulationsergebnisse für die gleiche Reihenfolge der Sequenzen, wie sie auch den Simulationen von Fig. 5A und 5B zugrunde lag, während die punktierten Linien die Simulationsergebnisse für eine andere Reihenfolge darstellen. Die Fig. 6A und 6B zeigen, dass die langsam variierende CR-Stimulation im Vergleich zur schnell variierenden CR-Stimulation robuster gegenüber der Reihenfolge der Sequenzen ist.

Die Simulationen zeigen darüber hinaus, dass auch die Anfangsbedingungen des Netzwerks Einfluss auf die Desynchronisation der neuronalen Aktivität haben. In den Fig. 6C und 6D ist jeweils die 50. Perzentile der Werte für den Synchronisationsgrad S für die letzten 16 Sekunden der Stimulation als eine Funktion der Stimulationsstärke K für zwei verschiedene Anfangsbedingungen des Netzwerks dargestellt. Die durchgezogenen Linien zeigen die Simulationsergebnisse für die gleichen Anfangsbedingungen des Netzwerks, wie sie auch den Simulationen von Fig. 5A und 5B zugrunde lagen, während die strichpunktierten Linien die Simulationsergebnisse für andere Anfangsbedingungen des Netzwerks darstellen. Auch hier zeigt sich die langsam variierende CR-Stimulation robuster gegenüber den Anfangsbedingungen des Netzwerks im Vergleich zur schnell variierenden CR-Stimulation.

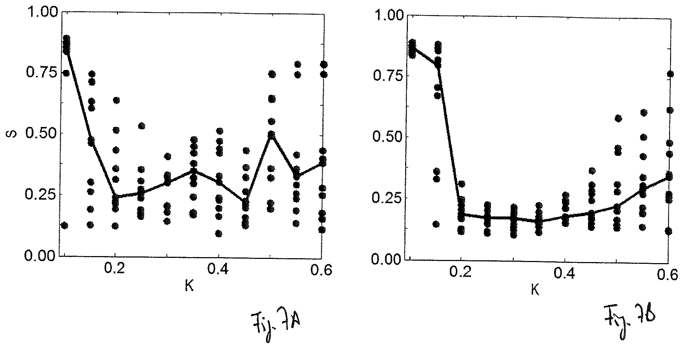

Sowohl für die schnell variierende als auch die langsam variierende CR-Stimulation wurden 10 weitere Simulationen mit unterschiedlichen Reihenfolgen der Sequenzen sowie unterschiedlichen Anfangsbedingungen des Netzwerks durchgeführt. Die Ergebnisse dieser Simulationen sind in den Fig. 7A und 7B gezeigt, in denen die 50. Perzentile der Werte für den Synchronisationsgrad S für die letzten 16 Sekunden der Stimulation als eine Funktion der Stimulationsstärke K für verschiedene Reihenfolgen der Sequenzen und verschiedene Anfangsbedingungen der Netzwerks aufgetragen ist. Der Median der Ergebnisse für jeden Wert der Stimulationsstärke K ist jeweils durch eine Linie verbunden. Fig. 7A zeigt die Ergebnisse der schnell variierenden CR-Stimulation, und Fig. 7B zeigt die Ergebnisse der langsam variierenden CR-Stimulation. Als Ergebnis lässt sich den Fig. 7A und 7B entnehmen, dass die langsam variierende CR-Stimulation das hoch synchrone neuronale Netzwerk stärker und robuster desynchronisiert, als dies mit der schnell variierenden CR-Stimulation möglich ist.

**Patentansprüche**

1. Vorrichtung (1; 2) zur Stimulation von Neuronen (31), umfassend

   - eine in den Körper eines Patienten implantierbare Stimulationseinheit (11) mit einer Mehrzahl von Stimulationselementen (12-15) zur Stimulation von Neuronen (31) im Gehirn und/oder Rückenmark (30) des Patienten mit Reizen (22), und
   - eine Steuereinheit (10), welche die Stimulationseinheit (11) derart ansteuert, dass die Stimulationselemente (12-15) Sequenzen von Reizen (22) repetitiv erzeugen,

   **dadurch gekennzeichnet, dass**

   - die Steuereinheit (10) die Stimulationseinheit (11) ferner derart ansteuert, dass die Reihenfolge, in welcher die Stimulationselemente (12-15) innerhalb einer Sequenz die Reize (22) erzeugen, für mindestens 20 nacheinander generierte Sequenzen konstant ist und danach variiert wird.

2. Vorrichtung (1; 2) nach Anspruch 1, wobei die Sequenzen in einem Zeitraster, das aus aufeinanderfolgenden Zyklen besteht, erzeugt werden und zumindest in einigen der Zyklen jeweils eine Sequenz von Reizen (22) erzeugt wird.

3. Vorrichtung (1; 2) nach Anspruch 2, wobei innerhalb eines jeweiligen Zyklus entweder genau eine Sequenz von Reizen (22) erzeugt wird oder keine Reize erzeugt werden.

4. Vorrichtung (1; 2) nach Anspruch 2 oder 3, wobei während n aufeinanderfolgenden Zyklen Reize (22) erzeugt werden und während der darauffolgenden m Zyklen keine Reize erzeugt werden und dieses Muster periodisch fortgesetzt wird, wobei n und m nicht-negative ganze Zahlen sind.

5. Vorrichtung (1; 2) nach einem der vorhergehenden Ansprüche, wobei das Muster, nach welchem die Reihenfolge, in welcher die Stimulationselemente (12-15) innerhalb einer Sequenz die Reize (22) erzeugen, für mindestens 20 nacheinander generierte Sequenzen konstant ist und danach die Reihenfolge variiert wird, mehrfach wiederholt wird.

6. Vorrichtung (1; 2) nach einem der vorhergehenden Ansprüche, wobei die Stimulationseinheit (11) L Stimulationselemente (12-15) enthält und in einer jeweiligen Sequenz von Reizen (22) P der L Stimulationselemente (12-15) Reize (22) erzeugen, wobei $L \geq 2$ und $2 \leq P \leq L$ gilt.

7. Vorrichtung (1; 2) nach Anspruch 6, wobei jedes der P Stimulationselemente (12-15) innerhalb einer jeweiligen Sequenz von Reizen (22) genau einen Reiz (22) erzeugt.

**8.** Vorrichtung (1; 2) nach Anspruch 7, wobei genau ein Reiz (22) genau ein elektrischer und/oder optischer Pulszug ist.

**9.** Vorrichtung (2) nach einem der vorhergehenden Ansprüche, umfassend eine Messeinheit (16) zum Aufnehmen von Messsignalen (23), die eine neuronale Aktivität der mit den Reizen (22) stimulierten Neuronen (31) wiedergeben.

**10.** Vorrichtung (2) nach Anspruch 9, wobei die Steuereinheit (10) derart ausgelegt ist, dass sie die Anzahl der nacheinander generierten Sequenzen, in denen die Reihenfolge der die Reize (22) erzeugenden Stimulationselemente (12-15) konstant ist, verlängert, wenn die Steuereinheit (10) anhand der Messsignale (23) feststellt, dass ein Synchronisationsgrad der stimulierten Neuronen (31) durch die Applikation der Reize (22) nicht um mindestens einen vorgegebenen Schwellwert reduziert wird.

**11.** Vorrichtung (1; 2) nach einem der vorhergehenden Ansprüche, wobei die Reize (22) elektrische und/oder optische Reize (22) sind.

**12.** Software zum Ausführen in einem Datenverarbeitungssystem, wobei die Software

- Steuersignale zum Ansteuern einer in den Körper eines Patienten implantierten Stimulationseinheit (11) erzeugt, wobei die Stimulationseinheit (11) eine Mehrzahl von Stimulationselementen (12-15) aufweist und die Stimulationselemente (12-15) Reize (22) zur Stimulation von Neuronen (31) im Gehirn und/oder Rückenmark (30) des Patienten erzeugen, wobei die Steuersignale die Stimulationseinheit (11) derart ansteuern, dass die Stimulationselemente (12-15) Sequenzen von Reizen (22) repetitiv erzeugen,

**dadurch gekennzeichnet, dass**

- die Steuersignale die Stimulationseinheit (11) ferner derart ansteuern, dass die Reihenfolge, in welcher die Stimulationselemente (12-15) innerhalb einer Sequenz die Reize erzeugen, für mindestens 20 nacheinander generierte Sequenzen konstant ist und danach variiert wird.

## Claims

**1.** An apparatus (1; 2) for stimulating neurons (31), comprising

- a stimulation unit (11) which is implantable into a patient's body, said stimulation unit having a plurality of stimulation elements (12-15) for stimulating neurons (31) in the patient's brain and/or spinal cord (30) with stimuli (22), and
- a control unit (10) controlling the stimulation unit (11) such that the stimulation elements (12-15) repetitively generate stimulus sequences (22), **characterized in that**
- the control unit (10) further controls the stimulation unit (11) such that the order in which the stimulation elements (12-15) generate the stimuli (22) within a sequence remains constant for at least 20 successively generated sequences and is then being varied.

**2.** The apparatus (1; 2) according to claim 1, wherein the sequences are generated with a time pattern consisting of successive cycles and wherein one stimulus sequence (22) is generated in at least some of the cycles.

**3.** The apparatus (1; 2) according to claim 2, wherein either exactly one stimulus sequence (22) is generated or no stimuli are generated during one respective cycle.

**4.** The apparatus (1; 2) according to claim 2 or 3, wherein stimuli (22) are generated during n successive cycles and no stimuli are generated during the subsequent m cycles, wherein this pattern is continued periodically, wherein n and m are non-negative integers.

**5.** The apparatus (1; 2) according to any one of the preceding claims, wherein the pattern, according to which the order in which the stimulation elements (12-15) generate the stimuli (22) within a sequence remains constant for at least 20 successively generated sequences and is then being varied, is repeated for a number of times.

**6.** The apparatus (1; 2) according to any one of the preceding claims, wherein the stimulation unit (11) contains L stimulation elements (12-15) and in a respective stimulus sequence (22) P of the L stimulation elements (12-15) generate stimuli (22), wherein $L \geq 2$, and $2 \leq P \leq L$ applies.

7. The apparatus (1; 2) according to claim 6, wherein each of the P stimulation elements (12-15) generates exactly one stimulus (22) within a respective stimulus sequence.

8. The apparatus (1; 2) according to claim 7, wherein exactly one stimulus (22) is exactly one electrical and/or optical pulse train.

9. The apparatus (2) according to any one of the preceding claims, comprising a measuring unit (16) for recording measuring signals (23) which represent a neuronal activity of the neurons (31) stimulated with the stimuli (22).

10. The apparatus (2) according to claim 9, wherein the control unit (10) is configured such that the number of successively generated sequences in which the order of the stimulation elements (12-15) generating the stimuli (22) remains constant is extended when the control unit (10) determines, on the basis of the measuring signals (23), that a degree of synchronization of the stimulated neurons (31) is not being reduced by at least one predetermined threshold value by applying the stimuli (22).

11. The apparatus (1; 2) according to any one of the preceding claims, wherein the stimuli (22) are electrical and/or optical stimuli (22).

12. A software for execution in a data processing system, wherein the software

- generates control signals for controlling a stimulation unit (11) implanted into a patient's body, wherein the stimulation unit (11) comprises a plurality of stimulation elements (12-15) and the stimulation elements (12-15) generate stimuli (22) for stimulating neurons (31) in the patient's brain and/or spinal cord (30), wherein the control signals control the stimulation unit (11) such that the stimulation elements (12-15) repetitively generate stimulus sequences (22),

**characterized in that**

- the control signals further control the stimulation unit (11) such that the order in which the stimulation elements (12-15) generate the stimuli within a sequence remains constant for at least 20 successively generated sequences and is then being varied.

**Revendications**

1. Dispositif (1 ; 2) pour la stimulation de neurones (31), comprenant

- une unité de stimulation (11) implantable dans le corps d'un patient, comportant une pluralité d'éléments de stimulation (12-15) pour la stimulation par excitations (22) de neurones (31) dans le cerveau et/ou la moelle épinière (30) du patient, et
- une unité de commande (10), laquelle commande l'unité de stimulation (11) de sorte que les éléments de stimulation (12-15) génèrent des séquences d'excitations (22) de manière répétée,

**caractérisé en ce que**

- l'unité de commande (10) commande en outre l'unité de stimulation (11) de sorte que l'ordre dans lequel les éléments de stimulation (12-15) génèrent les excitations (22) à l'intérieur d'une séquence est constant pendant au moins 20 séquences générées successivement, puis est modifié.

2. Dispositif (1 ; 2) selon la revendication 1, dans lequel les séquences sont générées dans une trame temporelle constituée de cycles successifs, et au moins une séquence d'excitations (22) est générée dans quelques-uns desdits cycles.

3. Dispositif (1 ; 2) selon la revendication 2, dans lequel soit une seule séquence d'excitations (22), soit aucune excitation n'est générée à l'intérieur d'un cycle respectif.

**4.** Dispositif (1 ; 2) selon la revendication 2 ou 3, dans lequel les excitations (22) sont générées au cours de n cycles successifs, et aucune excitation n'est générée au cours de m cycles suivants, et ce motif est périodiquement reproduit, dans lequel n et m sont des nombres entiers non négatifs.

**5.** Dispositif (1 ; 2) selon l'une des revendications précédentes, dans lequel le motif suivant lequel l'ordre où les éléments de stimulation (12-15) génèrent les excitations (22) à l'intérieur d'une séquence est constant pendant au moins 20 séquences générées successivement avant que l'ordre soit modifié, est répété plusieurs fois.

**6.** Dispositif (1 ; 2) selon l'une des revendications précédentes, dans lequel l'unité de stimulation (11) comprend L éléments de stimulation (12-15) et dans une séquence d'excitations (22) respective, P des L éléments de stimulation (12-15) génèrent des excitations (22), dans lequel $L \geq 2$ et $2 \leq P \leq L$.

**7.** Dispositif (1 ; 2) selon la revendication 6, dans lequel chacun des P éléments de stimulation (12-15) génère seulement une excitation (22) à l'intérieur d'une séquence d'excitations (22) respective.

**8.** Dispositif (1 ; 2) selon la revendication 7, dans lequel une seule excitation (22) est précisément un train d'impulsions électrique et/ou optique.

**9.** Dispositif (2) selon l'une des revendications précédentes, comprenant une unité de mesure (16) pour l'enregistrement de signaux de mesure (23) reproduisant une activité neuronale des neurones (31) stimulés par les excitations (22).

**10.** Dispositif (2) selon la revendication 9, dans lequel l'unité de commande (10) est prévue de manière à accroître le nombre de séquences générées successivement, dans lesquelles l'ordre des éléments de stimulation (12-15) générant les excitations (22) est constant, lorsque l'unité de commande (10) constate sur la base des signaux de mesure (23) qu'un degré de synchronisation des neurones (31) stimulés par application des excitations (22) de diminue pas d'au moins une valeur seuil prescrite.

**11.** Dispositif (1 ; 2) selon l'une des revendications précédentes, dans lequel les excitations (22) sont des excitations électriques et/ou optiques (22).

**12.** Logiciel destiné à être exécuté dans un système de traitement de données, dans lequel le logiciel

- génère des signaux de commande pour la commande d'une unité de stimulation (11) implantée dans le corps d'un patient, dans lequel l'unité de stimulation (11) comporte une pluralité d'éléments de stimulation (12-15) et les éléments de stimulation (12-15) génèrent des excitations (22) pour la stimulation de neurones (31) dans le cerveau et/ou la moelle épinière (30) du patient, dans lequel les signaux de commande commandent l'unité de stimulation (11) de sorte que les éléments de stimulation (12-15) génèrent des séquences d'excitations (22) de manière répétée,

**caractérisé en ce que**

- les signaux de commande commandent en outre l'unité de stimulation (11) de sorte que l'ordre dans lequel les éléments de stimulation (12-15) génèrent les excitations à l'intérieur d'une séquence est constant pendant au moins 20 séquences générées successivement, puis est modifié.

Fig. 1

Fig. 2A

Fig. 2B

Fig.3

Fig.4

Fig. 5A

Fig. 5B

Fig. 6A

Fig. 6B

Fig. 6C

Fig. 6D

Fig. 7A

Fig. 7B

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102010016461 A1

- US 20060069415 A1

**In der Beschreibung aufgeführte Nicht-Patentliteratur**

- **P. TEMPERLI ; J. GHIKA ; J.-G. VILLEMURE ; P. BURKHARD ; J. BOGOUSSLAVSKY ; F. VINGER-HOETS.** How do parkinsonian signs return after discontinuation of subthalamic DBS?. *Neurology,* 2003, vol. 60, 78
- **P. A. TASS ; L. QIN ; C. HAUPTMANN ; S. DOVEROS ; E. BEZARD ; T. BORAUD ; W. G. MEISSNER.** Coordinated reset neuromodulation has sustained after-effects in parkinsonian monkeys. *Annals of Neurology,* 2012, vol. 72, 816-820
- **I. ADAMCHIC ; C. HAUPTMANN ; U. B. BARNIKOL ; N. PAWELCYK ; O.V. POPOVYCH ; T. BARNIKOL ; A. SILCHENKO ; J. VOLKMANN ; G. DEUSCHL ; W. MEISSNER.** Coordinated Reset Has Lasting Aftereffects in Patients with Parkinson's Disease. *Movement Disorders,* 2014, vol. 29, 1679

- **P. A. TASS.** Transmission of stimulus-locked responses in two coupled phase oscillators. *Phys. Rev. E,* 2004, vol. 69, 051909-1, 24
- **N. E. HUANG et al.** The empirical mode decomposition and the Hilbert spectrum for nonlinear and non-stationary time series analysis. *Proc. R. Soc. A: Math. Phys. Eng. Sci.,* 1998, vol. 454, 903-995
- **N. E. HUANG et al.** A confidence limit for the empirical mode decomposition and Hilbert spectral analysis. *Proceedings of the Royal Society of London Series A,* 2003, vol. 459, 2317-2345